# EUROPEAN PATENT APPLICATION

(11) **EP 1 813 167 A1**
(43) Date of publication of application: **01.08.2007**
(21) Application number: 05770690.5
(22) Date of filing: 10.08.2005
(51) Int. Cl.: A45D 44/22, A61K 8/02, A61K 8/00, A61Q 19/00, D04H 1/04, D04H 1/42, D04H 1/46

(54) **SKIN COVERING SHEET FOR COSMETIC PREPARATION IMPREGNATION AND PROCESS FOR PRODUCING THE SAME, AND FACE MASK USING SAID SHEET**

(30) Priority: 11.08.2004 JP 2004234674
(71) Applicant: DAIWABO CO. LTD., Osaka-shi, Osaka 541-0056 (JP); The Procter and Gamble Company, Cincinnati, Ohio 45202 (US)
(72) Inventor: MAKIHARA, Hiroko, Kobe-shi, Hyogo 655-0035 (JP); OHNISHI, Kazuyuki, Takaishi-shi, Osaka 592-0003 (JP)
(74) Representative: HOFFMANN EITLE
(86) International application number: PCT/JP2005/014642
(87) International publication number: WO 2006/016601

(57) **Abstract**

A skin application sheet for being impregnated with a cosmetic preparation is provided which is suitable for a face mask and so on, and causes less irritation to skin, and has good application properties. A splittable conjugate fiber web containing at least 50% by mass of splittable conjugate fibers is placed on one surface or both surfaces of a hydrophilic nonwoven containing at least 50% by mass of hydrophilic fibers and hydroentanglement treatment is conducted to split the splittable conjugate fibers into ultrafine fibers with a fineness of 0.5dtex and to form a ultrafine fiber layer containing at least 10% by mass of the ultrafine fibers and to integrate the hydrophilic nonwoven and the ultrafine fiber layer so that a sheet is obtained. This sheet is impregnated with the cosmetic preparation and used in such a manner that the ultrafine fiber layer is a skin contact surface.

## Description

### Technical Field

The present invention relates to a skin application sheet for being impregnated with a cosmetic preparation which sheet is to be impregnated with a cosmetic preparation such as a moisturizing component, a cleansing component, an antiperspirant component, a fragrance component, a whitening component, a blood circulation-improving component, an ultraviolet rays protective component, and a slimming component, and is to be used by being applied to human skin. Further, the present invention relates to a face mask which is impregnated with liquid cosmetic preparation.

### Background Art

In the past, a face application cosmetic sheet such as a face mask which is impregnated with a cosmetic preparation has been used. A nonwoven which consists mainly of cotton has been used for a conventional sheet impregnated with a cosmetic preparation. This is because cotton has water absorbency and is suitable for being impregnated with a cosmetic preparation. However, the cotton nonwoven has slightly rough and hard feel and can irritate the user when it contacts with the skin. Further, Japanese Patent Application Laid-open No. 2003-93152 proposes a cosmetic preparation impregnated sheet wherein a fiber structural body is impregnated with a cosmetic preparation and the fiber structural body is formed of an inner layer of a fiber assembly containing hydrophobic fibers as a main component (specifically a polypropylene (PP) thermobonded nonwoven, a PP spunbonded nonwoven, a polyethylene terephthalate (PET) spunbonded nonwoven, or a PP/polyethylene (PE) air-through nonwoven), and top and bottom outer layers of fiber assemblies containing hydrophilic fibers as the main component.

### Disclosure of Invention

### Problem to be Solved by the Invention

As described above, various sheets impregnated with cosmetic preparations have been proposed or used. The demand for such sheets is currently increasing, and in conjunction, there is demand for sheets impregnated with cosmetic preparations which have better feel when used. Specifically, as previously described, there is demand that these sheets cause less irritation to the user when contacting with the skin, and have better application properties to the face. Herein, the "application properties" are evaluated by the length of time for which a sheet remains adhered to skin. Generally, since a sheet impregnated with a cosmetic preparation contains a large amount of liquid at the beginning of use, it easily adheres to the skin. As time passes, however, the sheet may be out of touch with the skin due to evaporation of the liquid. The application properties are said to be better when the time until this "out of touch" occurs is longer.

An object of the present invention is to provide a skin application sheet for being impregnated with a cosmetic preparation which causes less irritation to the skin and has better application properties to the skin, as well as a face mask using this sheet.

### Means to Solve Problems

Through investigations to solve these problems, the present inventors have learned that a skin application sheet for cosmetic preparation impregnation, which has good application properties to the skin and causes less irritation to the skin while maintaining a cosmetic preparation-impregnation ability, can be obtained by using a layered structure for the cosmetic preparation impregnation sheet, wherein the surface of the sheet and the inside of the sheet are made of particular fiber structures respectively, thus achieving the present invention. In other words, the skin application sheet for being impregnated with a cosmetic preparation of the present invention is a complex nonwoven wherein an ultrafine fiber layer which includes at least 10 mass% of ultrafine fibers with a fineness of at most 0.5 dtex and is placed on one surface or both surfaces of a hydrophilic fiber layer which includes at least 50 mass% of hydrophilic fibers, and these layers are integrated. The skin application sheet for being impregnated with a cosmetic preparation of the present invention (hereinafter it is shortened to "application sheet" or "sheet") is characterized in that the ultrafine fiber layer is overlaid on and integrated with the hydrophilic fiber layer, the ultrafine fiber layer occupies at least one surface of the nonwoven, and the sheet is used in such a manner that the ultrafine fiber layer is a skin contact surface.

One aspect of the skin application sheet for being impregnated with a cosmetic preparation of the present invention is a layered sheet including an ultrafine fiber layer which is formed by splitting splittable conjugate fibers and is positioned on one or both surfaces of a hydrophilic fiber layer which contains at least 50 mass% of hydrophilic fibers, wherein the hydrophilic fiber layer and the ultrafine fiber layer are integrated, and the ratio of splittable conjugate fibers included in the ultrafine fiber layer prior to the integration is at least 50 mass%. In the case where the ultrafine fiber layer is formed by splitting the splittable conjugate fibers, the surface of the layered sheet after the integration has a dense and smooth region, and the penetrated cosmetic preparation forms a uniform thin liquid film in this region, ensuring good application properties to the skin. The reason why the application sheet of the present invention is identified by defining the ratio of the splittable conjugate fibers before being split is that, as will be discussed later, when the ultrafine fibers are formed by splitting the splittable conjugate fibers, determining the ratio of the ultrafine fibers may be difficult in some situations. If the ratio of the ultrafine fibers can be determined, a sheet wherein the ratio of ultrafine fiber layer formed by splitting is at least 10 mass% is of course included in the sheet of the present invention, regardless of the ratio of the splittable conjugate fibers included in the ultrafine fiber layer prior to the integration.

In the application sheet of the present invention, the hydrophilic fiber layer and the ultrafine fiber layer are preferably integrated by fiber entanglement caused by hydroentanglement. In the sheet integrated by the hydroentanglement, a dense ultrafine fiber layer is formed and a soft feel can be achieved and a moderate stretchability and air permeability are ensured.

The application sheet of the present invention may have a pattern. This pattern is preferably created during integrating the hydrophilic fiber layer and the ultrafine fiber layer by the hydroentanglement. The application sheet having the pattern may have a pattern such as a diamond pattern, a triangle pattern, a circle pattern, a wave pattern or a square pattern, which is formed by regular repetition of a combination of a high fiber-density portion and a low fiber-density portion, and/or a combination of a portion having a large sheet thickness and a portion having a small sheet thickness. The sheet having such a pattern has excellent design effect. Further, the sheet having such a pattern has concavities and convexities in the sheet surface, and therefore, compared to a sheet with a flat surface, the level of adhesion due to the cosmetic preparation is low when the sheets impregnated with the cosmetic preparation overlap each other by folding the sheet. Generally, a skin application sheet for being impregnated with a cosmetic preparation is supplied being impregnated with the cosmetic preparation and folded up, and therefore must be unfolded when used. The folded sheet wherein the sheets are adhered to each other by the cosmetic preparation therebetween may be usually difficult to be unfolded because of unnecessary dripping of the cosmetic preparation. This fact may put annoyance on the user. Since the sheet having the pattern can be opened up more easily than the sheet having no pattern, causing less annoyance to the user, the sheet having the pattern can provide a product which can be quickly applied to the skin after opening.

The skin application sheet for being impregnated with a cosmetic preparation of the present invention is composed of the nonwoven with the structure as described above. Therefore, the present invention also provides a nonwoven for a skin application sheet for being impregnated with a cosmetic preparation.

The skin application sheet for being impregnated with a cosmetic preparation of the present invention is produced by a production method including placing a splittable conjugate fiber web containing at least 50 mass% of splittable conjugate fibers onto one or both surfaces of a hydrophilic nonwoven containing at least 50 mass% of hydrophilic fibers, and effecting hydroentanglement treatment so as to split the splittable conjugate fibers to form an ultrafine fiber layer which includes ultrafine fibers with a fineness of at most 0.5dtex and integrate the hydrophilic nonwoven and the ultrafine fiber layer. This production method makes it possible to form the ultrafine fiber layer and to integrate the hydrophilic nonwoven and the ultrafine fiber layer at the same time by the action of high-pressure water streams. Further, this production method makes it possible to entangle the ultrafine fibers in the ultrafine fiber layer to give a structure which has a dense and smooth ultrafine fiber layer in the surface.

A face mask of the present invention is characterized in that the application sheet of the present invention is impregnated with a liquid cosmetic preparation at a ratio in a range of from 200 mass parts to 2000 mass parts for 100 mass parts of the application sheet, and the ultrafine fiber layer is to be a skin contact surface.

### Effect of Invention

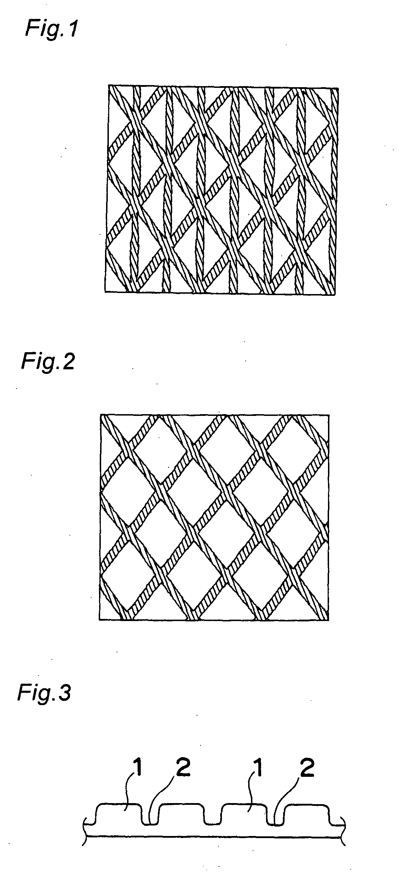

The application sheet of the present invention is constructed so that a part or all of the dense layer which is formed by entangling the ultrafine fibers with water streams is to contact with the skin. The entire of this dense layer provides a smooth surface when the application sheet has no pattern and is flat. In the case where the application sheet has the pattern, the convexities of the concaves and convexities formed by the pattern (that is, the portions which mainly contact the skin, and the portions indicated by the numeral "1" in Fig. 3) provide a dense and smooth surface. When such a sheet is impregnated with the liquid cosmetic preparation, the liquid cosmetic preparation forms a thin film on the smooth surface. This thin film of liquid is interposed between the skin and the application sheet to reduce the irritation (scratchy feel) to the skin. Further, the smooth surface provided by the ultrafine fiber layer itself causes less irritation to the skin and provides pleasant feel. Low irritation to the skin can be ensured by the formation of the dense and smooth surface where the ultrafine fibers are entangled in the portions which contacts with the skin (for example, the convexities of the concavities and convexities) even if the pattern is formed on the surface of the application sheet and the whole application sheet has the concavities and convexities. Therefore, the skin application sheet of the present invention further reduces the irritation (scratchy feel) to the skin of the user by synergistic effect of the interposition of the liquid thin film between the skin and the application sheet and the pleasant feel provided by the smooth and dense fiber layer. Furthermore, it can be said that the structure of the application sheet of the present invention easily forms a thin film of liquid cosmetic preparation on the sheet surface, and therefore the sheet of the present invention improves the skin application properties by virtue of the viscosity of the cosmetic preparation. In addition, since the application sheet of the present invention can be provided in a form where stretchability is high and an elongation modulus strength is low in one direction, the sheet can be adhered well to the skin when the sheet is applied to a specific location while being pulled slightly.

In the production method of the present invention, the hydrophilic fiber is first formed into a nonwoven, and then splittable conjugate fiber web is overlaid and then a hydroentanglement treatment is conducted. This production method can make the sheet surface smoother and suppress exposure of the hydrophilic fibers on the sheet surface compared with a production method wherein the splittable conjugate fiber web is overlaid on the hydrophilic fiber web and then the hydroentanglement treatment is conducted. Thereby, the application sheet with less irritation to the skin can be obtained. Furthermore, the ultrafine fiber is formed by the technique of splitting the splittable conjugate fibers with the hydroentanglement treatment in this production method. This also contributes to the increase in denseness of the sheet surface and reduces the irritation of the resultant application sheet to the skin. In addition, when the application sheet of the present invention has high stretchability and a low modulus strength in one direction and the face mask is constructed such that the one direction corresponds to the lateral direction of the face, the application of the mask to the face can be smoothly performed and the adhesion of the mask to the face becomes better.

The face mask of the present invention wherein the application sheet of the present invention is impregnated with a liquid cosmetic preparation is well adhered to the face and is not liable to come unstuck from the face and gives the skin less irritation due to the scratchy feel of fibers, because of the flexibility of the application sheet and the viscosity of the thin film of the liquid cosmetic preparation formed on the surface of the application sheet. Therefore, the face mask of the present invention enables the specified active ingredients in the liquid cosmetic preparation to be effectively applied to the skin without giving unpleasant feel to the user.

### Brief Description of Drawings

Fig. 1 is a plan view schematically showing an embodiment of an application sheet of the present invention having a pattern.
Fig. 2 is a plan view schematically showing another embodiment of the application sheet of the present invention having a pattern.
Fig. 3 is a sectional view schematically showing an embodiment of the application sheet of the present invention having a pattern.

### Description of Preferred Embodiments

The skin application sheet for being impregnated with a cosmetic preparation of the present invention includes a hydrophilic fiber layer and an ultrafine fiber layer as described above. The hydrophilic fiber layer in the application sheet of the present invention contains at least 50 mass% of hydrophilic fiber. If the hydrophilic fiber content is less than 50 mass%, the amount of the hydrophilic fibers in the overall application sheet is small and the pickup of the liquid cosmetic preparation is small. The amount of the hydrophilic fibers in the hydrophilic fiber layer is preferably at least 80 mass%. The form of the hydrophilic fiber layer before being integrated with the ultrafine fiber layer is a nonwoven or a fiber web, and preferably a nonwoven. The hydrophilic fiber may be a natural fiber such as pulp, cotton, linen, silk, and wool; a reproduced fiber such as viscous rayon, cuprammonium rayon, and a solvent spinning cellulose fiber; and a synthetic fiber subjected to a hydrophilicizing treatment. The hydrophilic fiber layer is formed from any one or more fibers selected from these fibers.

The hydrophilic fiber content is preferably within a range of from 10 mass parts to 70 mass parts for 100 mass parts of the application sheet. A more preferable hydrophilic fiber content is within a range of from 15 mass parts to 50 mass parts for 100 mass parts of the application sheet. If the hydrophilic fiber content is less than 10 mass parts, the pickup of the liquid cosmetic preparation may be small, and if the content is greater than 70 mass parts, excessive liquid cosmetic preparation may penetrate into the hydrophilic fiber layer, making it difficult to form the aforementioned thin film of the cosmetic preparation on the surface of the application sheet. Furthermore, if the ratio of the hydrophilic fibers occupying the sheet is large, the hydrophilic fibers may be exposed on the surface of the sheet, resulting in the increase in irritation given by the application sheet to the skin.

The hydrophilic fiber layer is generally the fiber web and or the nonwoven before being integrated with the ultrafine fiber layer. The fiber webs include, for example, carded webs such as a parallel web, a cross-laid web, a semirandom-laid web and a random-laid web, an airlaid web, a wetlaid web, and a spunlaid web. The nonwovens are formed by using these fiber webs, and include, for example a thermobonded nonwoven such as a air-through nonwoven and a heat rolled nonwoven wherein the carded web or the airlaid web obtained by mixing the hydrophilic fibers with binder fibers are thermally bonded by the binder fibers, a wetlaid nonwoven wherein the fibers of the wetlaid web are entangled or bonded (including a tissue obtained by bonding pulp with a binder, or a tissue obtained by bonding pulp using hydrogen bonding), a needlepunched nonwoven wherein the fibers in the fiber web are entangled by needlepunching, and a hydroentangled nonwoven wherein the fibers in the fiber web are entangled by high-pressure water streams. If the hydrophilic nonwoven is used as the hydrophilic fiber layer, the surface of the application sheet obtained by integrating the hydrophilic layer with the ultrafine fiber layer can be easily made smooth and dense and therefore a thin layer of the liquid cosmetic preparation is easily formed on the sheet surface, as compared to the sheet wherein the hydrophilic fiber web is used.

The hydrophilic fiber layer is preferably in a form of the wetlaid nonwoven before being integrated with the ultrafine fiber. This is because the wetlaid nonwoven has a high fiber density (a fiber occupancy rate per unit volume). In general, as the fiber density of the nonwoven is higher (in other words, the nonwoven is denser), a denser structure can be achieved in the surface of the ultrafine fiber layer of the sheet that is obtained by integrating the nonwoven and the ultrafine fiber layer, resulting in a smoother surface. Therefore, when the wetlaid nonwoven is used as the hydrophilic fiber layer, the application sheet can be obtained which has better application properties. The wetlaid nonwoven may be a rippled or creased one, so-called a crepe paper, so long as the nonwoven does not impair the smoothness of the surface of the resultant sheet which surface will particularly contacts with the skin.

The hydrophilic fiber layer is more preferably a wetlaid nonwoven which includes pulp fibers, prior to being integrated with the ultrafine fiber layer. The pulp fiber is preferably used because it has a flat fiber cross-section and the wetlaid nonwoven which contains the pulp fibers form a construction wherein flat faces are stacked when hydroentanglement is conducted, and this gives, to the sheet, a construction where the hydrophilic fibers are not readily exposed to the surface of the application sheet and therefore the sheet causes less irritation to the skin. The wetlaid nonwoven including the pulp fibers may be formed of 100 mass% of pulp fibers, or may be one wherein the pulp fibers are bonded together with a binder or hydrogen bond between pulp. The wetlaid nonwoven wherein the pulp fibers are wetlaid is generally called as a tissue paper, and as will be discussed later, the tissue paper is preferable because of the excellent entanglement with splittable conjugate fiber web when integrated with this web by the hydroentanglement treatment.

The mass per unit area of the hydrophilic fiber layer is preferably between 7g/m² and 50g/m². More preferably, the mass per unit area of the hydrophilic fiber layer is between 10g/m² and 40g/m², and even more preferably between 10g/m² and 30g/m². If the mass per unit area of the hydrophilic fiber layer is less than 7g/m², the liquid cosmetic preparation may not be sufficiently retained. If the mass per unit area of the hydrophilic fiber layer is greater than 50g/m², there may be a decrease in entanglement with the ultrafine fiber layer positioned on at least one of the surfaces of the hydrophilic fiber layer.

In the case where the hydrophilic fiber layer takes a nonwoven form before being integrated with the ultrafine fiber layer, the nonwoven density of the nonwoven (that is, the hydrophilic nonwoven) under a load of 2.94cN/cm² is preferably between 0.05g/cm³ and 0.3g/cm³, more preferably between 0.05g/cm³ and 0.2g/cm³, and even more preferably between 0.06g/cm³ and 0.15g/cm³. If the nonwoven density is below 0.05g/cm³, excessive liquid cosmetic material may be retained inside the application sheet and a thin film of the liquid cosmetic preparation may be difficult to form on the surface of the application sheet. If the density of the nonwoven is greater than 0.3g/cm³, there may be a tendency that the air permeability of the nonwoven is impaired and an application sheet produced using this type of nonwoven may cause the user to have a feeling of pressure when applied to the skin.

In the case where the hydrophilic fiber layer takes the nonwoven form before being integrated with the ultrafine fiber layer, this nonwoven (that is, the hydrophilic nonwoven) preferably has from 10cm³/cm²·sec to 80cm³/cm²·sec of the air permeability. More preferably, the air permeability is between 15cm³/cm²·sec and 50cm³/cm²·sec. If the air permeability is less than 10cm³/cm²·sec, the user may have a feeling of pressure when the resultant application sheet is applied to the skin. If the air permeability is greater than 80cm³/cm²·sec, there may be a disadvantage that excessive liquid cosmetic preparation is retained inside the application sheet because the interfiber spaces are large. Furthermore, if the air permeability (that is, the interfiber spaces) of the hydrophilic nonwoven is large, excessive fiber entanglement may occur during the hydroentanglement process for integrating the hydrophilic nonwoven and the ultrafine fiber layer, whereby the smoothness of the sheet surface may be impaired, and as a result, a thin-film of the liquid cosmetic preparation may be difficult to form on the sheet surface.

The hydrophilic nonwoven which satisfies the aforementioned range for nonwoven density and/or air permeability is preferably the wetlaid nonwoven produced by a wetlaying method, which nonwoven includes at least one type of fiber selected from a natural fiber such as pulp, cotton linter, and linen, and a reproduced fiber such as viscous rayon, cuprammonium rayon and a solvent spinning cellulose fiber.

The hydrophilic fiber layer which takes the nonwoven form before being integrated with the ultrafine fiber layer has been primarily described above. With the application sheet of the present invention, the hydrophilic fiber layer is not necessarily required to be the nonwoven. For example, a carded web of the reproduced fibers as it is may also be integrated with the ultrafine fiber layer.

The fiber length of the hydrophilic fiber included in the hydrophilic fiber layer is not limited to particular one, and is optimally selected depending on the form of the hydrophilic fiber layer before being integrated with the ultrafine fiber layer. However, if the hydrophilic fibers are long, the hydrophilic fibers may be exposed on the surface of the resultant application sheet, resulting in more irritation to the skin. For this reason, the fiber length of the hydrophilic fiber is preferably at most 100mm. More specifically, when the hydrophilic fiber layer is a fiber carded web or a nonwoven of the carded web, the fiber length is preferably selected from a range of from 20mm to 70mm, and when the hydrophilic fiber layer is a wetlaid web or a wetlaid nonwoven made from such a wetlaid web, the fiber length is preferably selected from a range of from 2mm to 20mm.

Next, the ultrafine fiber layer which is overlaid onto at least one surface of the hydrophilic fiber layer is described. The ultrafine fiber layer contains at least 10 mass% of the ultrafine fibers with a fineness of at most 0.5dtex. The ultrafine fiber may be formed using, for example, a meltblown method, and in this case, the ultrafine fiber layer is a meltblown nonwoven. In the present invention, the ultrafine fiber is preferably made by splitting a splittable conjugate fiber. This is because the splittable conjugate fibers make it possible to form the ultrafine fibers and entangle the ultrafine fibers tightly by the high-pressure water streams, giving a dense ultrafine fiber layer.

The fineness of the ultrafine fiber is required to be 0.5 dtex or less in both of the case where the ultrafine fibers are formed using the meltblown method and the case where the ultrafine fibers are formed by splitting the splittable conjugate fibers. More preferable fineness of the ultrafine fiber is 0.4dtex or less. If the fineness of the ultrafine fiber is greater than 0.5dtex, the liquid cosmetic preparation may be retained in the interfiber spaces of the surface of the application sheet, and the thin film of the liquid cosmetic preparation may be difficult to form on the surface of the application sheet. It is noted that the lower limit for the fineness of the ultrafine fiber is not limited to particular one, but the fineness is preferably 0.05 dtex or greater, considering the abrasion resistance of the application sheet surface.

In the case where the splittable conjugate fibers are used, the splittable conjugate fibers preferably comprises at least 50 mass% of the ultrafine fiber layer prior to the hydroentanglement treatment, and more preferably comprises at least 80 mass%. In the ultrafine fiber layer after the hydroentanglement treatment, the splittable conjugate fibers do not need to be completely split into the respective structural components. For example, splitting only a part of the structural components is acceptable. Alternatively, it is also acceptable that the ultrafine fiber is not a completely independent fiber and a single or a plurality of ultrafine fibers branch off of a single splittable conjugate fiber. In the case where the splitting of the splittable conjugate fiber into the ultrafine fibers is stopped halfway, determining the ratio of the ultrafine fibers in the ultrafine fiber layer may be difficult. It is should be noted that, in consideration of this, the application sheet of the present invention is identified using the ratio of the splittable conjugate fibers prior to the hydroentanglement. In this case, the cross-section of the sheet can be observed under a microscope or the like to determine whether or not the ultrafine fibers are contained in an amount sufficient to achieve the desired effect of the present invention. For instance, when the sheet is cut along parallel to the width direction of the sheet and enlarged 300 times with an electron microscope so that the cross section of the ultrafine fiber layer including the skin contact surface is observed and then over 10 or more strands of the ultrafine fibers are observed in a region of [400µm in the widthwise direction of the sheet] x [200µm in the depth direction of the sheet], the sheet can accomplish the desired effect as the sheet of the present invention.

The splittable conjugate fiber used in the present invention has a fiber cross-section structure wherein at least one component is divided into two or more segments, and at least part of each component is exposed to the fiber surface, and the exposed portion is continuously formed in the longitudinal direction of the fiber. Combinations of preferable polymers which make up the splittable conjugate fibers include polyethylene terephthalate/polyethylene, polyethylene terephthalate/polypropylene, polyethylene terephthalate /ethylene propylene copolymer, polypropylene/polyethylene, and polyethylene terephthalate/nylon and so on. If the splittable conjugate fiber is used, the fiber length thereof is selected from a range of, for example, from 5mm to 100mm depending on the web form employed in the production method described below.

The ultrafine fiber which makes up the ultrafine fiber layer is preferably a hydrophobic fiber with a standard moisture regain of at most 5%. In the case where the ultrafine fiber is a hydrophobic fiber, the fiber itself does not absorb the liquid cosmetic preparation, and therefore the thin film of liquid cosmetic preparation is more easily formed on the surface of the application sheet. Herein, the standard moisture regain refers to one after the fiber is made. However, since the standard moisture regain of a synthetic fiber does not change significantly before and after forming the fiber, the standard moisture regain of the resin prior to being formed into a fiber may be used as the standard moisture regain for the fiber without modification unless a hydrophilicizing process is conducted in the fiber producing process. When the ultrafine fiber is formed by splitting the splittable conjugate fiber, a hydrophobic ultrafine fiber can be obtained by using the splittable conjugate fiber with a combination such as polyethylene terephthalate/polyethylene, polyethylene terephthalate/polypropylene, polyethylene terephthalate/ethylene propylene copolymer, or polypropylene/polyethylene.

The mass per unit area of the ultrafine fiber layer (or the mass per unit area of each ultrafine fiber layer if the ultrafine fiber layers are placed on both surfaces of the hydrophilic fiber layer) is preferably between 15g/m² and 50g/m². A more preferable mass per unit area of the ultrafine fiber layer is between 20g/m² and 40g/m². If the mass per unit area of the ultrafine fiber layer is less than 15g/m², the thickness of the ultrafine fiber layer may be thin, and therefore there may be a tendency that the hydrophilic fibers are exposed to the surface, resulting in increase in irritation to the skin. If the mass per unit area of the ultrafine fiber layer is greater than 50g/m², the entire application sheet may have a high mass per unit area to give feeling of pressure to the user when applied to the skin.

Other fibers may also be mixed with the hydrophilic fibers and the ultrafine fibers in the hydrophilic fiber layer and the ultrafine fiber layer respectively to the degree that the effect of the present invention is not hindered. For example, the ultrafine fiber layer may contain up to 30 mass% of hydrophilic fibers. The hydrophilic fiber may be, for example, a regenerated fiber such as viscous rayon, cuprammonium rayon, and a solvent spinning cellulose fiber. If the hydrophilic fiber is included in the ultrafine fiber layer, the sheet is more easily impregnated with the liquid cosmetic preparation. Furthermore, the application sheet of the present invention may also include other fiber layers (such as a nonwoven or a sheet-like material) as long as the effect of the present invention is not hindered. However, even if other fiber layers are included, the ultrafine fiber layer is required to be located on at least one surface side of the application sheet and the surface of the ultrafine fiber layer is required to be able to contact with the skin.

The skin application sheet for being impregnated with a cosmetic preparation of the present invention is a complex nonwoven wherein the ultrafine fiber layer is overlaid on at least one surface of the hydrophilic fiber layer, and these layers are integrated. The integration may be accomplished by a bonding technique using an adhesive or a thermal bonding conjugate fiber consisting of a combination of a low melting-point resin and a high melting-point resin, or by a fiber-entanglement technique such as needlepunching or hydroentanglement. The integration of the hydrophilic fiber layer and the ultrafine fiber layer is preferably realized by the fiber entanglement with the hydroentanglement. The reason for this is, as described above, that a dense ultrafine fiber layer, and good softness and air permeability are achieved. In particular, when the ultrafine fiber layer is the splittable conjugate fiber web, the hydroentanglement is preferably used as the integration technique. The reason for this is that the uniform and dense structure is realized in the sheet surface because the splittable conjugate fibers are split to form the ultrafine fibers and the entanglement of the ultrafine fibers proceeds during the hydroentanglement.

The complex nonwoven preferably has a mass per unit area which is within the aforementioned preferred range for each of the hydrophilic fiber layer and the ultrafine fiber layer, and has an entire mass per unit area within the range of from 22g/m² to 150g/m², more preferably within the range of from 50g/m² to 120g/m², and even more preferably within the range of from 60g/m² to 110g/m². If the mass per unit area of the complex nonwoven is small, the effect conferred by the ultrafine fiber layer and/or the hydrophilic fiber layer may be insufficient, and if the mass per unit area is large, the sheet may give feeling of pressure to the user when applied to the skin.

The application sheet of the present invention obtained as the complex nonwoven in this manner, preferably has high stretchability in one direction, and low stretchability in a direction orthogonal to the one direction. The stretchability is expressed as the degree of elongation (that is, an elongation percentage) of the sheet at breaking. If the application sheet of the present invention is used as, for example, the face mask, the application sheet is preferably cut such that the direction of high stretchability corresponds to the lateral direction of the face and the direction of low stretchability corresponds to the longitudinal direction of the face. In the case where an application sheet with different levels of stretchability in the longitudinal and lateral directions is used as the face mask, the mask can be adhered well to the recesses and protrusions such as eyes, nose and mouth or the like by locally stretching the sheet when applied to the face, resulting in improvement in the application properties to the face. The direction with high stretchability is generally the lateral direction (CD direction) of the complex nonwoven. Specifically, the application sheet of the present invention preferably has an elongation percentage (that is, the rupture elongation when stretched in this direction) between 120% and 250% in the direction of high stretchability. When the elongation percentage is within this range, and the application sheet of the present invention is used as the face mask in particular, the application sheet can be comfortably applied to the recesses and protrusions such as the eyes, the nose, and the mouth or the like of the face. The application sheet of the present invention more preferably has the elongation percentage between 130% and 200%, still more preferably greater than 140% and at most 200%, and most preferably between 150% and 200% in the direction of high stretchability.

The application sheet of the present invention preferably also has a low modulus strength in one direction and a high modulus strength in the direction orthogonal to the one direction in which the sheet has low modulus strength. Herein, the modulus strength refers to the force necessary to elongate a sheet by a prescribed amount. For the present invention, the modulus strength is evaluated by the forces necessary to achieve a 10% elongation and a 20% elongation in a test sample of 5cm width (these forces are referred to as the "10%-elongation modulus strength" and the "20%-elongation modulus strength"). If the application sheet of the present invention is used as, for example, the face mask, the application sheet is preferably cut such that the direction of low modulus strength corresponds to the lateral direction of the face. The low 10%-elongation modulus strength and the low 20%-elongation modulus strength mean that the force necessary in the initial steps of elongating the sheet is small. Generally, when the face mask is applied, the user tends to apply the mask while extending the mask in the lateral direction of the face in order to achieve close contact of the mask to the skin. When other regions are covered by the sheet, the sheet is normally applied while being elongated in one direction of the sheet. Therefore, if the resistance is low when elongating the sheet, a product with a better feel during use can be provided. The study by the present inventors has shown that the force necessary to achieve a 10% to 20% elongation affects the feel during use of the sheet (particularly, the face masks).

Specifically, the application sheet of the present invention preferably has a 10%-elongation modulus strength of between 0.3N/5cm and 3N/5cm, or a 20%-elongation modulus strength of between 0.5N/5cm and 5N/5cm. The 20%-elongation modulus is more preferably between 0.7N/5cm and 4.5N/5cm. The direction in which the sheet has a low modulus strength generally corresponds to the aforementioned direction of high stretchability, and this direction is generally the lateral direction (CD direction) of the complex nonwoven. If the 10%-elongation modulus is less than 0.3N/5cm or if the 20%-elongation modulus strength is less than 0.5N/5cm, the sheet may be stretched even under minute forces, which makes the handling of the sheet difficult. If the 10%-elongation modulus strength is greater than 3N/5cm, or if the 20%-elongation modulus strength is greater than 5N/5cm, the sheet may be difficult to stretch, whereby achieving good adhesion to the skin may be difficult.

Furthermore, the application sheet of the present invention obtained as the complex nonwoven preferably has from 40cm³/cm²·sec to 130cm³/cm²·sec of the air permeability. The air permeability of the complex nonwoven is more preferably between 50cm³/cm²·sec and 125cm³/cm²·sec, and even more preferably between 50cm³/cm²·sec and 110cm³/cm²·sec. If the air permeability of the complex nonwoven is less than 40cm³/cm²·sec, the user may have a feeling of pressure when the sheet is applied to the skin. If the air permeability of the complex nonwoven is greater than 130cm³/cm²·sec, the penetrated liquid cosmetic preparation may be easily retained inside the complex nonwoven, and there is a tendency that the thin film of the liquid cosmetic preparation is difficult to form on the surface of the complex nonwoven. It should be noted that the hydrophilic nonwoven used to manufacture the application sheet of the present invention may have an air permeability which is lower than that of the final complex nonwoven. In other words, the air permeability of the complex nonwoven may be higher than that of the hydrophilic nonwoven. It is considered that this is caused by the fact that the fibers of the hydrophilic nonwoven are rearranged by the effect of the energy of the water streams when the hydrophilic nonwoven and the ultrafine fiber layer are integrated by the hydroentanglement. For instance, when a wetlaid nonwoven containing pulp fibers is used as the hydrophilic fiber layer and integrated with the ultrafine fiber layer by the hydroentanglement, the pulp fibers are scattered in the regions where the water streams strike because of the short fiber length of the pulp fibers, whereby the regions where the pulp fibers are scattered have fewer fibers and therefore have the high air permeability. As a result, the air permeability of the entire complex nonwoven generally has a tendency to be higher than that of a wetlaid nonwoven. It should be noted that the air permeability of the entire complex nonwoven is required to be adjusted within an appropriate range, considering this tendency.

The application sheet of the present invention which is obtained as the complex nonwoven may also have a pattern. The pattern may be formed by any method such as an embossing roller or a hydroentanglement treatment with an appropriate supporting member. The pattern is preferably formed by the hydroentanglement treatment. This is because, according to the hydroentanglement treatment, the dense and smooth surface is formed in the convexities of the concavities and convexities formed by the pattern and the entire sheet has the softness and the stretchability suitable for the skin covering. It is particularly preferable that the hydroentanglement treatments is performed in two steps, wherein the hydrophilic fiber layer and the ultrafine fiber layer are integrated to make the sheet dense to some degree by the first treatment and then the pattern is formed by the later hydroentanglement treatment. When such a two-step hydroentanglement treatment is carried out, the dense structure formed earlier is maintained after forming the pattern. For this reason, this treatment technique is advantageous in that the surface of the convexities of the concavities and convexities forming the pattern is made dense and smooth.

The pattern is preferably formed by placing a fiber web onto a supporting member which has an appropriate opening pattern based on the desired pattern, and then carrying out the hydroentanglement so that portions where the fibers are coarse and portions where the fibers are thick, and/or portions where the sheet thickness is thin and portions where the thickness is thick are regularly arranged. The specific method for forming the pattern is described below. Examples of the patterns are schematically shown in Figs. 1 to 3. Figs. 1 and 2 are plan views schematically showing examples of the pattern formed in the skin application sheet of the present invention, and Fig. 3 is a sectional view of the sheet having the pattern. In Figs. 1 and 2, the portions where the fiber density is low are hatched for easy comprehension. The application sheet with the pattern generally has the concavities and convexities wherein the dense portions are the convexities (1) and the coarse portions are the concavities (2). The application sheet which has the convexities and concavities is advantageous in that it is easily unfolded after being impregnated with the cosmetic preparation and then folded over as described above. Further, the application sheet with the pattern tends to have a higher air permeability than that without the pattern. Therefore, the face mask manufactured using the sheet having the pattern is suitable for covering the face for longer periods of time and suitable for other applications for which high air permeability is desired.

The pattern preferably has a dimension and a shape such that the convexities account for between 40% and 90% of the whole surface of the sheet. If the ratio of the convexities is low, the area of the application sheet which contacts with the skin is small and thereby good application properties may not be obtained. If the ratio of the convexities is large, the effect of the concavities and the convexities may not be sufficiently obtained. A step between the concavity and the convexity is preferably between 0.01mm and 0.8mm. If the step is too large, the application properties to the skin may deteriorate. Further, the convexities are preferably formed such that the number thereof is between 3 and 20 every 10cm in the longitudinal direction of the sheet, and the number thereof is between 3 and 20 every 10cm in the lateral direction. If each convexity is small, the concavities and convexities may be finer to provide undesired feel on the skin. However, depending on the production method, the application sheet having the pattern may have the concavities and convexities with a large step on one surface and be relatively flat on the other surface. In that case, the flat surface may be used as the skin contact surface so long as the flat surface is the surface of the ultrafine fiber layer. When this usage embodiment is employed, the step between the convexity and the concave may have an insignificant effect on the feel and the application properties, and therefore the ratio and/or the number of the convexities may be outside the aforementioned range.

More specific dimensions are exemplified below. For example, in the pattern shown in Fig. 1, each convexity may be an isosceles triangle wherein the base is between about 10mm and 16mm and the height is between about 3mm and 6mm, and each concave may have a width between about 1mm and about 3mm. In the pattern shown in Fig. 2, each convexity may be a diamond shape wherein the length of one side is between 5mm and 10mm and the combinations of two adjacent sides form an obtuse angle between 100° to 110° and an acute angle between 70° to 80°.

The application sheet of the present invention obtained in the form of the complex nonwoven is impregnated with the liquid cosmetic preparation and used in such a manner that the ultrafine fiber layer is the skin contact surface. By making the ultrafine fiber layer as the skin contact surface, irritation to the skin can be reduced and application properties to the skin can be improved. When the ultrafine fiber layer is placed on only one surface of the hydrophilic fiber layer, for instance, all or a part of any one of the surfaces is preferably colored, printed, or marked with a mark, so that the surface of the ultrafine fiber layer can be identified. The construction wherein the ultrafine fiber layer is placed on both surfaces of the hydrophilic fiber layer is advantageous in that the user can use the sheet without worrying about the front and back.

Further, as described above, when an application sheet having the pattern is relatively flat on one surface, the application sheet is preferably used such that the flat surface is the skin contact surface. Therefore, in that case as well, all or part of this flat surface is preferably colored, printed, or marked with a mark in order to be easily identified by the user.

Next, a preferred production method of the skin application sheet for being impregnated with a cosmetic preparation of the present invention is described. First, the hydrophilic nonwoven which is to be the hydrophilic fiber layer in the final application sheet is prepared. As previously mentioned, the hydrophilic nonwoven may be any one of nonwovens which are produced by a conventional method using the hydrophilic fibers. The preferable mass per unit area and the preferable air permeability or the like of the hydrophilic nonwoven have been described previously, and therefore the description thereof is omitted here.

Next, the fiber web which is to be the ultrafine fiber layer in the final application sheet is prepared. In this production method, a splittable conjugate fiber web which includes at least 50 mass% of splittable conjugate fibers is prepared for forming the ultrafine fiber layer. The splittable conjugate fiber web may have any form selected from a carded web such as a parallel web, a cross-laid web, a semirandom-laid web, or a random-laid web, an airlaid web, a wetlaid web, and a spunlaid web or the like. The splittable conjugate fiber web is preferably a carded web in view of irritation to the skin.

The order of preparation of the hydrophilic nonwoven and the splittable conjugate fiber web is not limited to particular one, and it is acceptable to prepare the splittable conjugate fiber web first. Alternatively, both layers may be prepared simultaneously in parallel, or it is also acceptable to prepare the hydrophilic nonwoven, prepare the splittable conjugate fiber web, and conduct the hydroentanglement treatment to be discussed later on a single line.

The splittable conjugate fiber web is placed on one or both surfaces of the prepared hydrophilic nonwoven, and then hydroentanglement is performed so that the fibers are entangled and the hydrophilic nonwoven and the splittable conjugate fiber web are integrated. Furthermore, during the hydroentanglement treatment, the splittable conjugate fibers are split to form the ultrafine fibers and the ultrafine fibers are entangled with each other. The hydroentanglement treatment may be performed according to a conventional method and the conditions are optimally selected depending on the mass per unit area of the application sheet to be obtained and the desired splitting rate of the splittable conjugate fibers and so on. The hydroentanglement treatment may be performed by placing the web on a 80- to 100-mesh plain weave supporting member, and spraying the water streams with a water pressure of at least 1MPa and at most 10MPa on the splittable conjugate fiber web side (or on both sides if the splittable conjugate fiber web is positioned on both sides) once to four times using a nozzle with orifices having a diameter between 0.05mm and 0.5mm at an interval between 0.3mm and 1.5mm. In particular, it is preferable that hydroentanglement is performed at a low water pressure of less than 7MPa because the exposure of the hydrophilic fibers on the surface of the base sheet can be suppressed, and the resultant application sheet causes little irritation to the skin. After the hydroentanglement, the water is removed by drying, and as a result, the skin application sheet for being impregnated with a cosmetic preparation of the present invention or the nonwoven for the skin application sheet for being impregnated with a cosmetic preparation of the present invention can be obtained.

The application sheet having a pattern is obtained by placing a fiber web on a fine plain weave supporting member (for example, 80- to 100-mesh), spraying on the splittable conjugate fiber web side with the water streams at a water pressure between 1MPa and 10MPa from a nozzle having the orifices of the aforementioned diameter at the aforementioned interval, and then placing the supporting member which has holes in order to create the desired pattern, on a fine plain weave supporting member (for example, 80 to 100 mesh), and then placing the web on the supporting member having the holes, and spraying the water streams at a water pressure between 1MPa and 10MPa on the side opposite to the side which is to have a large step between the concavity and the convexity which are formed by the pattern (that is, the side where the pattern is more clearly expressed) from the nozzle having the orifices of the aforementioned diameter at the aforementioned interval. With this method, the surface of the resultant sheet which surface is on the side where the water streams are sprayed becomes relatively flat. Alternatively, the pattern may be formed by a method where the web is placed on the fine plain weave supporting member, a supporting member with holes is then placed on top of this web, and the water streams are sprayed toward the supporting member with holes, or a method wherein the web is placed on a supporting member in which a plate with holes for forming the pattern is attached to the fine plain weave supporting member, and then water streams are sprayed. Alternatively, a supporting member which is made by forming holes or protrusions without holes in a nonporous metal plate or a porous metal plate that has a plurality of small holes may be used, and the web may be placed on the supporting member and the pattern may be formed by spraying the water streams. In this case as well, the surface of the resultant sheet which surface is on the side where the water streams are sprayed becomes relatively flat. The supporting members or plates for creating the pattern including the embodiments described above, are generally constructed by regularly forming openings and/or recessions with or without holes. The openings and/or recessions have shapes corresponding to the dense portions and/or the convexities in the application sheet. Such a supporting member or plate is produced by a conventional method and therefore the detailed description thereof is omitted here.

The application sheet of the present invention can be produced using a method other than the method described above. For instance, the application sheet of the present invention can be obtained by preparing a fiber web which contains the hydrophilic fibers as the hydrophilic fiber layer without forming a nonwoven, placing a splittable conjugate fiber web on one or both surfaces of the web, and then conducting hydroentanglement treatment. Alternatively, the application sheet of the present invention may also be produced by a method wherein a meltblown web (or a meltblown nonwoven) made of the ultrafine fibers is used in place of the splittable conjugate fiber web and overlaid on at least one surface of the hydrophilic nonwoven or the hydrophilic fiber web, and then hydroentanglement treatment is performed.

Alternatively, the application sheet of the present invention may be produced by a method without employing the hydroentanglement treatment. For example, the hydrophilic fiber layer and the ultrafine fiber layer (which previously contains the ultrafine fibers formed by splitting the splittable conjugate fibers when the splittable fibers are used) may be provided as a sheet such as a nonwoven and these layers may be integrated using an adhesive. Alternatively, thermal bonding fibers may be mixed into the hydrophilic fiber layer and/or the ultrafine fiber layer and these fiber layers may be integrated by stacking these layers and then heating the stack so that the thermal bonding fibers are melted or softened. Alternatively, the hydrophilic fiber layer and the ultrafine fiber layer may be integrated by needlepunching. Alternatively, any of these integration methods and the hydroentanglement treatment may be combined to produce the application sheet of the present invention.

Next, the face mask of the present invention is described. The face mask of the present invention is made by impregnating 100 mass parts of the application sheet of the present invention with a liquid cosmetic preparation in an amount between 200 mass parts and 2000 mass parts, and more preferably between 200 mass parts and 1500 mass parts. The amount of the liquid cosmetic preparation within this range makes it possible to provide a sufficient amount of active ingredients to the skin, and to avoid inconvenience (such as liquid dripping) during use. The optimum amount of the liquid cosmetic preparation is appropriately determined by the properties of the application sheet, and particularly by the water absorbency of the sheet. In a preferred embodiment, the amount of the liquid cosmetic preparation is adjusted so that the liquid cosmetic preparation exists in an amount equal to or greater than the saturation amount of the application sheet during the predetermined duration of use. The liquid cosmetic preparation includes a water-soluble thickener and a water-based carrier, and the type and the amount of the water-soluble thickener are preferably adjusted so that the viscosity becomes between 500mPa·sec and 60,000mPa·sec.

The face mask of the present invention is used to keep the ultrafine fiber layer in contact with the face. The face mask is formed in the shape which is suitable for covering the face, and for example, may have punched regions or incised regions, if necessary, corresponding to the eyes, the nose, and the mouth. Alternatively, the face mask may be formed to cover only a part of the face (such as the area around the eyes, area around the mouth, or the nose, or cheeks). Alternatively, the face mask may be provided as a set comprising a sheet which covers the area around the eyes and a sheet which covers the area around the mouth, or may be provided as a set of sheets which cover three or more areas respectively. These sets which are comprised of two or more sheets simplify the whole face covering with a sheet. The liquid cosmetic preparation preferably contains as an active ingredient, for example, a moisture retaining component, a cleansing component, an antiperspirant component, a perfume component, a whitening component, a blood-circulation improving component, an ultraviolet rays protective component, or a slimming component or the like, but it is not limited to any of these components, and it may contain any component which can be expected to provide a specific effect on the skin.

The application sheet for being impregnated with a cosmetic preparation of the present invention may be used for other applications other than face masks. For instance, the application sheet of the present invention impregnated with a liquid cosmetic preparation including a moisture retaining component may also be applied to the neck, elbow, or heel of the foot. Alternatively, the application sheet of the present invention may be impregnated with a liquid cosmetic preparation containing a slimming component and applied to the abdomen or thighs.

### Examples

The present invention is described below by examples.

### [Test sample 1]

A wetlaid nonwoven which was formed of 100 mass% of pulp fibers and had a mass per unit area of about 17g/m² (made by HAVIX CORPORATION) was prepared as a hydrophilic nonwoven. This wetlaid nonwoven had 0.113g/cm³ of the nonwoven density under a load of 2.94cN/cm². Furthermore, this wetlaid nonwoven had 2SCM3/CM2. sec of the air permeability.

On the other hand, two semirandom-laid carded webs were prepared as the splittable conjugate fiber webs. Each web had a mass per unit area of about 27g/m² and was made of 100 mass% of 8-section splittable conjugate fibers formed of polyethylene terephthalate (standard moisture regain 0.4%)/polyethylene (standard moisture regain 0%) which fibers had a fineness of 2.2dtex and a fiber length of 51mm. Next, the splittable conjugate fiber webs were placed on both sides of the hydrophilic nonwoven. The water streams were sprayed twice at a pressure of 3MPa and 6MPa respectively from a nozzle having orifices of a 0.1mm diameter with a 0.6mm interval on one surface of this layered fiber web, and then the water streams were sprayed at a pressure of 5MPa twice on the other surface, so that the fibers were entangled with each other to integrate the hydrophilic nonwoven and the splittable conjugate fiber webs. The spraying of the water streams was carried out placing the fiber web on and conveyed by a 90-mesh plain weave supporting member at a speed of 10m/min. Here, approximately 60 mass% of the splittable conjugate fibers which made up the splittable conjugate fiber web were at least partially split, and as a result, the ultrafine fiber layer containing the ultrafine fibers with a fineness of approximately 0.275dtex was formed. After the hydroentanglement treatment, the drying was conducted at 100°C to give a complex nonwoven as a skin application sheet for being impregnated with a cosmetic preparation. The sheet had 85cm³/cm²·sec of the air permeability. Further, the sheet was cut along a line parallel to the width direction and magnified 300 times using an electron microscope in order to observe the cross-section of the ultrafine fiber layer which includes the skin contact surface, and when observed, 25 strands of the ultrafine fibers were observed in the region [400µm in the lateral direction of the sheet] x [200µm in the thickness direction of the sheet].

### [Test sample 2]

A wetlaid nonwoven which was formed of 100 mass% of pulp fibers and had a mass per unit area of about 22g/m² (made by HAVIX CORPORATION) was prepared as the hydrophilic nonwoven. This wetlaid nonwoven had 0.12g/cm³ of the nonwoven density under a load of 2.94cN/cm². This wetlaid nonwoven had 20cm³/cm²·sec of the air permeability. The skin application sheet for being impregnated with a cosmetic preparation was obtained according to the same method as that for producing Test sample 1, except that the conveying speed of the fiber web was 4m/min during the spray of the water streams. Also in this sample, approximately 60 mass% of the splittable conjugate fibers which made up the splittable conjugate fiber web were at least partially split, and as a result, the ultrafine fiber layer containing the ultrafine fibers with a fineness of approximately 0.275dtex was formed. The sheet had 78cm³/cm²·sec of the air permeability. 25 Strands of the ultrafine fibers were observed by observing the section of the sheet in the same manner as in Test sample 1.

### [Test sample 3]

A hydroentangled nonwoven having a mass per unit area of 20g/m² was prepared as the hydrophilic nonwoven. The nonwoven was produced by' subjecting, to the hydroentanglement treatment, a semirandom-laid carded web which was formed of 100 mass % of rayon fibers having a fineness of 1.7dtex and a fiber length of 40mm (made by DAIWABO RAYON Co., Ltd., trade name CORONA). This rayon nonwoven had 0.042g/cm³ of the nonwoven density under a load of 2.94cN/cm² and 180cm³/cm²·sec of the air permeability. The skin application sheet for being impregnated with a cosmetic preparation was produced according to the same method as that for producing Test sample 1 except that this rayon nonwoven was used and the conveying speed was 4m/min during the spray of the water streams. Also in this sample, approximately 60 mass% of the splittable conjugate fibers which made up the splittable conjugate fiber web were at least partially split, and as a result, the ultrafine fiber layer containing the ultrafine fibers with a fineness of approximately 0.275dtex was formed. The sheet had 120cm³/cm²·sec of the air permeability. 25 Strands of the ultrafine fibers were observed by observing the section of the sheet in the same manner as in Test sample 1.

### [Test sample 4]

The skin application sheet for being impregnated with a cosmetic preparation was produced according to the same method as that for producing Test sample 1 except that a semirandom-laid carded web was prepared which was formed of 100 mass% of rayon fibers having a fineness of 1.7dtex and a fiber length of 40mm (made by DAIWABO RAYON Co., Ltd., trade name CORONA) and the splittable conjugate fiber webs were overlaid on both surfaces of the carded web and the conveying speed was 4m/min during the spray of the water streams. In this sample, approximately 50 mass% of the splittable conjugate fibers which made up the splittable conjugate fiber web were at least partially split, and as a result, the ultrafine fiber layer containing the ultrafine fibers with a fineness of approximately 0.275dtex was formed. The sheet had 142cm³/cm²·sec of the air permeability. 20 Strands of the ultrafine fibers were observed by observing the section of the sheet in the same manner as in Test sample 1.

### [Test sample 5]

A layered nonwoven was prepared as the hydrophilic nonwoven, which comprised of two layers of wetlaid nonwovens each of which was formed of 100 mass % of pulp fibers and had a mass per unit area of about 13.2g/m² (made by HAVIX CORPORATION). This layered wetlaid nonwoven had 0.18g/cm³ of the nonwoven density under a load of 2.94cN/cm² and 19cm³/cm²·sec of the air permeability. Further, the semirandom-laid carded web having a mass per unit area of about 38g/m² was prepared using the same fibers as those used in Test sample 1. The skin application sheet for being impregnated with cosmetic preparation was obtained according to the same method as that for producing Test sample 1, using these wetlaid nonwoven and fiber web. Also in this sample, approximately 60 mass% of the splittable conjugate fibers which made up the splittable conjugate fiber web were at least partially split, and as a result, the ultrafine fiber layer containing the ultrafine fibers with a fineness of approximately 0.275dtex was formed. The sheet had 65cm³/cm²·sec of the air permeability. 23 Strands of the ultrafine fibers were observed by observing the section of the sheet in the same manner as in Test sample 1.

### [Test sample 6]

The hydrophilic-fiber nonwoven and the splittable conjugate fiber web which were the same as those used for producing Test sample 1 were used to make a layered web and the water streams were sprayed on this web by the following procedures. First, the layered web was placed on a 90-mesh plain weave supporting member and the water streams were sprayed at a pressure of 3MPa on one surface of the web, using a nozzle having orifices of a 0.1mm diameter with a 0.6mm interval. The conveying speed was 10m/min. Next, a supporting member for forming the pattern as shown in Fig. 1 (which had holes corresponding to the portions of high fiber density (the convexities)) was placed on the 90-mesh plain weave supporting member and the hydroentangled layered web was placed on the supporting member for forming the pattern. Next, the water streams were sprayed at a pressure of 2MPa on one surface of the fiber web, using a nozzle having orifices of a 0.1mm diameter with a 0.6mm interval. The conveying speed was 10m/min. Next, the drying treatment was conducted on the same conditions as those employed in the production of Test sample 1. As a result, the application sheet for being impregnated with a cosmetic preparation was obtained, wherein one surface had a concavo-convex pattern of a diamond shape and the other surface was relatively flat. More specifically, the diamond pattern was one formed by combinations of the convexities each of which was an isosceles triangle having a base of about 16mm and a height of about 6mm. Also in this sample, approximately 60 mass% of the splittable conjugate fibers which made up the splittable conjugate fiber web were at least partially split, and as a result, the ultrafine fiber layer containing the ultrafine fibers with a fineness of approximately 0.275dtex was formed. The sheet had 119.2cm³/cm²·sec of the air permeability. 22 Strands of the ultrafine fibers were observed by observing the section of the sheet in the same manner as in Test sample 1.

The mass per unit area, the thickness and the density of the resultant each sample are shown in Table 1. Herein, the thickness was determined with a thickness meter (manufactured by Daiei Kagaku Seiki Seisakusho Co., Ltd., trade name: THICKNESS GAUGE model CR-60A) under a load of 3g per 1cm² of the sample. Further, Test samples 1 to 4 were subjected to a tensile test to determine the elongation percentage, 10% and 20% elongation modulus strengths, in which test a sample piece of 5cm in width was held at a grasp interval of 10cm and extended at a pulling rate of 30±2cm/min with a constant speed extension tensile tester (manufactured by ORIENTEC Co., LTD, trade name: TENSILON) in accordance with JIS L-1096 6.12.1 A (strip method). In the tensile test, the elongation direction was the direction of the nonwoven along which the nonwoven shows high stretchability, that is, the lateral direction (CD direction). The elongation percentage, and 10% and 20% elongation modulus strengths are shown in Table 1.

**Table 1**

| Test Sample | Mass per Unit Area (g/m²) | Thickness (mm) | Density (g/cm³) | Elongation Percentage (%) | 10% Elongation Modulus Strength (N/5cm) | 20% Elongation Modulus Strength (N/5cm) |
|---|---|---|---|---|---|---|
| 1 | 70 | 0.80 | 0.088 | 151.0 | 1.10 | 2.30 |
| 2 | 82 | 0.89 | 0.092 | 177.0 | 2.30 | 4.10 |
| 3 | 82 | 0.97 | 0.085 | 155.0 | 2.20 | 4.70 |
| 4 | 82 | 0.95 | 0.086 | 161.0 | 2.00 | 3.60 |
| 5 | 99 | 0.90 | 0.091 | 137.5 | 1.43 | 2.71 |
| 6 | 69 | 0.84 | 0.082 | 155.0 | 0.46 | 0.98 |

As shown in Table 1, all of Test samples 1 to 6 had a high elongation percentage in the lateral direction. Further, Test samples 1 to 6 had a 10% elongation modulus strength of at most 3N/5cm, and a 20% elongation modulus of at most 5N/5cm, and therefore could be stretched relatively easily with a small force.

The test samples obtained were stamped out into a shape of a face so that the direction of the nonwoven along which the high stretchability was shown corresponds to the lateral direction of the face, and incisions were made in the regions corresponding to eyes, mouth, and nose, to make the shape of a face mask. This mask was folded once along the longitudinal center line of the face (the line parallel to the ridge of the nose), and then folded another time along the longitudinal line which passes through the center, and then folded one more time along the horizontal line which passes through the center so that the sheet was folded in eight. The sheet which was folded in eight was put into an A6 size polyethylene bag with zipper, and 700 mass parts of a liquid cosmetic preparation with the composition shown in Table 2 for 100 mass parts of the sheet was also put into the polyethylene bag, and the zipper was sealed so as to prevent as much air as possible from entering. Next, the polyethylene bag containing the sheet and the liquid cosmetic preparation was placed on a desk and the bag was pressed five times with the palm of the hand, and then the zipper of the polyethylene bag was opened and the sheet was taken out.

**Table 2**

| Component | Wt% |
|---|---|
| Methyl paraben | 0.07 |
| Disodium EDTA | 0.05 |
| Sodium Salicylate | 0.5 |
| 1,3-butylene glycol | 10 |
| Xanthan rubber | 0.6 |
| De-ionized water | remainder |

With all of the test samples, the penetration status of the liquid cosmetic preparation was good. Furthermore, when the sheet impregnated with the liquid cosmetic preparation was applied to the face, there was little irritation to the skin, the sheet did not easily unstick after the application, and adhesion to the face was good.

As an indicator for expressing the ease of unfolding a sheet which has been impregnated with liquid, the determination was made as to the ratio (the area percentage) of the convexities which occupy the surface of the skin application sheet for being impregnated with a cosmetic preparation which had a pattern. Specifically, the dimension of a region was determined, which contained six convexities in the longitudinal direction of the nonwoven and six convexities in the lateral direction of the nonwoven was measured for Test sample 6. For Test sample 6, the dimension of the region was 12.6cm in the longitudinal direction x 9.1cm in the lateral direction. Next, the area of each of the convexities which were included in this region was measured, and the values were summed. The total area of the convexities was divided by the area of the region so as to obtain the area percentage of the convexities. The area percentage of the convexities in Test sample 6 was 75%. In actuality, Test sample 6 could easily be unfolded and quickly applied to the face.

### Industrial Applicability

The skin application sheet for being impregnated with a cosmetic preparation of the present invention was soft, and, when impregnated with the liquid cosmetic preparation, forms a thin liquid film on the surface to make a buffer layer, and therefore the sheet is suitable for being applied to every part of the human body, particularly sensitive parts of the body such as the whole face, a nose, an area around eyes, an area around mouth, and a neck or the like, and specifically, can be used as a face mask.

## Claims

1. A skin application sheet for being impregnated with a cosmetic preparation wherein an ultrafine fiber layer which comprises at least 10% by mass of ultrafine fibers with a fineness of at most 0.5dtex and is placed on one surface or both surfaces of a hydrophilic fiber layer which comprises at least 50% by mass of hydrophilic fiber, the hydrophilic fiber layer and the ultrafine fiber layer are integrated, and the ultrafine fiber layer is to be the surface which contacts with the skin.

2. A skin application sheet for being impregnated with a cosmetic preparation wherein an ultrafine fiber layer which comprises ultrafine fibers with a fineness of at most 0.5dtex formed by splitting splittable conjugate fibers is placed on one surface or both surfaces of a hydrophilic fiber layer which comprises at least 50% by mass of hydrophilic fibers, wherein the hydrophilic fiber layer and the ultrafine fiber layer are integrated, the ultrafine fiber layer is to be the surface which contacts with the skin, and the ultrafine fiber layer comprises at least 50% by mass of the splittable conjugate fibers before being integrated with the hydrophilic fiber layer.

3. The skin application sheet for being impregnated with a cosmetic preparation according to claim 1 or 2, wherein said hydrophilic fiber layer and said ultrafine fiber layer are integrated by hydroentanglement.

4. The skin application sheet for being impregnated with a cosmetic preparation according to claim 1 or 2, wherein said hydrophilic fiber layer comprises hydrophilic fibers at a ratio of at least 10 mass parts and at most 70 mass parts for 100 mass parts of the skin application sheet.

5. The skin application sheet for being impregnated with a cosmetic preparation according to claim 1 or 2, wherein said hydrophilic fiber layer is a wetlaid nonwoven comprising pulp fibers.

6. The skin application sheet for being impregnated with a cosmetic preparation according to claim 1 or 2, wherein said ultrafine fibers are hydrophobic fibers with a standard moisture regain of at most 5%.

7. The skin application sheet for being impregnated with a cosmetic preparation according to claim 1 or 2, wherein said skin application sheet has from 40cm³/cm²·sec to 130cm³/cm²·sec of an air permeability.

8. The skin application sheet for being impregnated with a cosmetic preparation according to claim 1 or 2 wherein said application sheet has a pattern which forms regular concavities and convexities.

9. A nonwoven for a skin application sheet for being impregnated with a cosmetic preparation comprising an ultrafine fiber layer which comprises at least 10% by mass of ultrafine fibers with a fineness of at most 0.5dtex and is placed on one surface or both surfaces of a hydrophilic fiber layer which comprises at least 50% by mass of hydrophilic fiber, wherein the hydrophilic fiber layer and the ultrafine fiber layer are integrated, and the ultrafine fiber layer is to be the surface which contacts with the skin.

10. A nonwoven for a skin application sheet for being impregnated with a cosmetic preparation comprising an ultrafine fiber layer which comprises ultrafine fibers with a fineness of at most 0.5dtex formed by splitting splittable conjugate fibers, and is placed on one surface or both surfaces of a hydrophilic fiber layer which comprises at least 50% by mass of hydrophilic fibers, wherein the hydrophilic fiber layer and the ultrafine fiber layer are integrated, the ultrafine fiber layer is to be the surface which contacts with the skin, and the ultrafine fiber layer comprises at least 50% by mass of the splittable conjugate fibers before being integrated with the hydrophilic fiber layer.

11. A method for producing a skin application sheet for being impregnated with a cosmetic preparation comprising:
placing a splittable conjugate fiber web comprising at least 50% by mass of splittable conjugate fibers onto one surface or both surfaces of a hydrophilic nonwoven which comprises at least 50% by mass of hydrophilic fibers; and
effecting hydroentanglement to form an ultrafine fiber layer comprising at least 10% by mass of ultrafine fibers with a fineness of at most 0.5dtex by splitting said splittable conjugate fibers and to integrate the hydrophilic nonwoven and the ultrafine fiber layer.

12. The method according to claim 11, wherein said hydrophilic nonwoven has from 0.05g/cm³ to 0.2g/cm³ of a nonwoven density under a load of 2.94cN/cm².

13. The method according to claim 11 or 12, wherein said hydrophilic nonwoven has from 10cm³/cm²·sec to 80cm³/cm²·sec of an air permeability.

14. A face mask comprising a skin application sheet for being impregnated with a cosmetic preparation according to claim 1 or 2 impregnated with a liquid cosmetic preparation at a ratio in a range of at least 100 mass parts and at most 2000 mass parts for 100 mass parts of the sheet.

15. The face mask according to claim 14, wherein said liquid cosmetic preparation comprises a water-soluble thickener and a water-based carrier.
